# EUROPÄISCHE PATENTSCHRIFT

(11) **EP 3 034 029 B1**
(45) Veröffentlichungstag und Bekanntmachung des Hinweises auf die Patenterteilung: **28.11.2018**
(21) Anmeldenummer: 15197681.8
(22) Anmeldetag: 03.12.2015
(51) Int. Cl.: A61B 90/70, A47L 15/50

(54) **SPÜLMASCHINE MIT EINEM SPÜLGUTTRÄGER**
DISHWASHER HAVING A WASHED OBJECT HOLDER
LAVE-VAISSELLE COMPRENANT UN PORTE-VAISSELLE

(30) Priorität: 18.12.2014 DE 102014119105
(43) Veröffentlichungstag der Anmeldung: 22.06.2016
(73) Patentinhaber: Miele & Cie. KG, 33332 Gütersloh (DE)
(72) Erfinder: Thorman, Franz, 33649 Bielefeld (DE)

(56) Entgegenhaltungen:
- EP-A1- 2 201 886
- EP-A2- 1 946 692
- EP-A2- 2 465 407

## Beschreibung

Die Erfindung betrifft eine Spülmaschine, insbesondere einen Reinigungs- und/oder Desinfektionsautomat für schlauchförmiges Reinigungsgut, mit einem zumindest eine Beschickungsöffnung aufweisenden Gehäuse (2) und einer die Beschickungsöffnung verschließenden Tür (3, 4), wobei das Gehäuse (2) und die geschlossene Tür (3, 4) zusammen einen geschlossenen Spülraum (5) bereitstellen, sowie mit einem Spülgutträger (6) zum Einschub in eine Spülmaschine (1), welcher Spülgutträger (22) in einer Einschubrichtung (7) relativ zum Gehäuse (2) bewegbar ist und einen Trägerkörper (22) zur Aufnahme von Spülgut aufweist, welcher sich in Einschubrichtung (7) erstreckende Längsseiten und rechtwinklig zur Einschubrichtung (7) erstreckende Querseiten (20, 21) aufweist.

Gattungsgemäße Spülgutträger und entsprechende Spülmaschinen sind aus dem Stand der Technik bekannt, beispielsweise aus der DE 10 2007 003 894 B4 und der EP 2 201 886 A1.

Die EP 2465407 A2 offenbart darüber hinaus eine Geschirrspülmaschine mit einer Besteckschublade, die mit einer beispielsweise als Feder ausgebildeten automatische Bewegungseinrichtung gekoppelt ist, welche dazu eingerichtet ist, die Besteckschublade bei geöffneter Tür automatisch teilweise aus dem Spülbehälter zu bewegen.

Eine Spülmaschine dient insbesondere dazu, Spülgut zu reinigen, gegebenenfalls zu desinfizieren und zu trocknen. In der besonderen Ausgestaltung als Reinigungs- und/oder Desinfektionsautomat für schlauchförmiges Reinigungsgut handelt es sich beispielsweise um Spülmaschinen, welche in medizinischen Einrichtungen zur Reinigung und Entkeimung medizinischer Gerätschaften wie Trokare und dergleichen verwendet.

Das zu behandelnde Spülgut wird unter Verwendung eines Spülgutträgers, auch Spülkorb genannt, im Spülraum der Spülmaschine angeordnet. Hierbei ist es bekannt, eine fluidtechnische Verbindung zwischen dem Gehäuse der Spülmaschine einerseits und dem Spülgutträger andererseits auszubilden, um beispielsweise Spülflüssigkeit, Desinfektionsflüssigkeit und/oder Trocknungsluft gezielt auf das zu reinigende Spülgut verteilen zu können. Um diese fluidtechnische Verbindung auszubilden, ist am Gehäuse der Spülmaschine eine Kupplungsvorrichtung und am Spülgutträger ein Anschlussstück vorgesehen.

Zur Ausbildung der fluidtechnischen Verbindung ist es erforderlich, dass die spülmaschinenseitige Kupplungsvorrichtung einerseits und das spülgutträgerseitige Anschlussstück andererseits lagegenau zueinander ausgerichtet sind. Der Vorschlag der DE 10 2007 003 894 B4 geht dahin, die beiden Teile unter Verwendung eines beweglichen Dorns miteinander zu verrasten. Der Vorschlag der EP 2 201 886 A1 geht hingegen dahin, das Anschlussstück lösbar an einer Seitenwand des Spülgutträgers anzuordnen.

Wenngleich sich die vorstehend beschriebenen Maßnahmen grundsätzlich bewährt haben, besteht dennoch der Bedarf an einer vereinfachten und zugleich noch lagegenaueren Anordnung des Spülgutträgers im Spülraum.

Vor diesem Hintergrund ist es die **Aufgabe** der vorliegenden Erfindung, den Spülgutträger mit vereinfachten Mitteln noch lagegenauer im Spülraum einer Spülmaschine anzuordnen.

Zur **Lösung** schlägt die Erfindung eine Spülmaschine mit den Merkmalen von Anspruch 1 vor.

Erfindungsgemäß ist folglich vorgesehen, den Spülgutträger mit einer Art federnden Anschlag auszustatten. Dieser Anschlag ist stirnseitig am Trägerkörper angeordnet. Er steht in Einschubrichtung über den Trägerkörper vor. Wird der Spülgutträger in Einschubrichtung gegen ein von der Spülmaschine bereitgestelltes Widerlager, beispielsweise eine den Spülraum der Spülmaschine begrenzende Rückwand oder Tür, geschoben, schlägt nicht der Trägerkörper selbst an demselben an, sondern vielmehr die Federeinrichtung.

Auf diese Weise lässt sich der Spülgutträger ohne das sonst übliche Vorsehen eines Sicherheitsabstands in Einschubrichtung im geschlossenen Spülraum einspannen. Dies bedeutet, dass der Spülgutträger in Einschubrichtung mit der Spülmaschine in Kontakt steht. Dieser Kontakt wird durch die erfindungsgemäß vorgesehene Federeinrichtung ermöglicht, da die Federeinrichtung für einen etwaig erforderlichen Ausgleich von

Toleranzen der Länge des geschlossenen Spülraums in Einschubrichtung sorgt.

Das Einspannen des Spülgutträgers in der Spülmaschine ermöglicht wiederum eine besonders lagegenaue Anordnung desselben. Weist die Spülmaschine beispielsweise eine feststehende Rückwand auf, kann der Trägerkörper des Spülgutträgers in direkten Kontakt mit der Rückwand gebracht werden. An der gegenüberliegenden Seite weist der Trägerkörper hingegen die Federeinrichtung auf, welche beim Verschließen der Tür an derselben anschlägt und etwaige Positionierungsungenauigkeiten der Tür durch Einfedern ausgleicht.

Derartige Positionierungsungenauigkeiten einer Tür können insbesondere daher rühren, dass die Dichtungen zwischen dem Gehäuse und der Tür im Laufe der Lebensdauer der Spülmaschine verschleißen und sich infolgedessen der Abstand zwischen der Tür und dem Gehäuse verändert. Dadurch ändert sich auch die Länge des geschlossenen Spülraums in Einschubrichtung.

Das erfindungsgemäße Konzept ist auch und besonders bei sogenannten Durchschubmaschinen von Vorteil. Hierbei handelt es sich um Spülmaschinen, insbesondere Reinigungs- und/oder Desinfektionsautomaten für schlauchförmiges Reinigungsgut, welcher zwei einander gegenüber angeordnete Beschickungsöffnungen aufweisen, welche jeweils mit einer Tür verschließbar sind. Gerade bei solchen Spülmaschinen sind die zuvor bereits thematisierten Kupplungseinrichtungen im Bereich der Seitenwand der Spülmaschine angeordnet, so dass eine lagegenaue Positionierung des Spülgutträgers besonders wichtig ist. Insbesondere bei der Verwendung einer Durchschubmaschine kann daher vorgesehen sein, dass bei dem Spülgutträger an beiden einander in Einschubrichtung gegenüber liegenden Querseiten jeweils stirnseitig eine Federeinrichtung angeordnet ist.

Nach dem Einschieben eines derartigen Spülgutträgers in den Spülraum und Verschließen desselben, ist der Spülgutträger zwischen den beiden Türen eingespannt. Die Federeinrichtungen an den beiden Querseiten bewirken, dass der Trägerkörper in eine Mittellage zwischen den beiden Türen drängt.

Durch die Federeinrichtungen können etwaige Toleranzen in Einschubrichtung auf beiden Seiten des Spülgutträgers ausgeglichen werden.Eine derartige Toleranz führt nämlich in erster Linie zu einer Auslenkung der Federeinrichtungen, nicht zu einer Änderung der Lage des Trägerkörpers im Spülraum. Der an beiden Querseiten mit Federeinrichtungen versehene Trägerkörper behält folglich seine Relativlage im Spülraum toleranzunabhängig bei.

Durch die Federeinrichtungen an beiden Querseiten des Trägerkörpers wird zudem eine Vorzentrierung des Trägerkörpers im Spülraum erreicht. Die Lage eines herkömmlichen, ohne Federeinrichtungen ausgebildeten Spülgutträgers im Spülraum lässt sich oftmals nur mit einer vergleichsweise großen Toleranz von etwa 1-2 cm vorgeben. Dies unter anderem deswegen, da immer ein gewisser Bewegungsspielraum des Spülgutträgers in Einschubrichtung verbleibt. Die Verbindung zwischen einer spülmaschinenseitigen Kupplungsvorrichtung einerseits und einem spülgutträgerseitigen Anschlussstück andererseits kann aber dann wesentlich zuverlässiger hergestellt werden, wenn der Spülgutträger mit einer vergleichsweise kleinen Toleranz von nur wenigen Millimetern genau positioniert werden kann. Die an beiden Querseiten des Trägerkörpers angeordneten Federeinrichtungen bewirken durch das beidseitige Anschlagen im geschlossenen Spülraum, dass der Spülgutträger bzw. der Trägerkörper im Rahmen seiner Bewegungsfreiheit nicht eine undefinierte Lage, sondern vielmehr durch die Federkräfte der Federeinrichtungen eine definierte, vorzentrierte Lage im Spülraum einnimmt. Die toleranzbedingten Abweichungen von dieser definierten, vorzentrierten Lage sind wesentlich geringer als die zuvor thematisierten 1-2 cm, so dass gegenüber herkömmlichen Spülgutträgern eine wesentlich zuverlässige Verbindung zwischen der Kupplungsvorrichtung und dem Anschlussstück erreicht werden kann.

Vorzugsweise sind die beiden Federeinrichtungen gleich ausgebildet. Sie weisen die gleichen Federeigenschaften, insbesondere die gleiche Federkennlinie auf. Nimmt man im Falle einer Durchschubmaschine beispielsweise an, dass sich die Dichtungen beider Türen in etwa gleichmäßig abnutzen, ist die damit einhergehende Abstandsänderung zwischen Gehäuse und Tür an beiden Türen im Wesentlichen gleich. Der an beiden Querseiten mit gleich ausgebildeten Federeinrichtungen versehene Trägerkörper behält in diesem Falle seine Relativlage im Spülraum konstant bei, da eine gleichmäßige Verringerung des Abstands zwischen Tür und Gehäuse auf beiden Seiten durch entsprechend gleichmäßiges Einfedern beider Federeinrichtungen an beiden Seiten ausgeglichen wird. Der so ausgestaltete Spülgutträger kann auch als im geschlossenen Spülraum schwimmend gelagert bezeichnet werden.

Die Erfindung bringt den Vorteil einer besonders einfachen und dennoch präzisen Positionierung des Spülgutträgers, insbesondere des Trägerkörpers, im Spülraum mit sich. Sofern der Spülgutträger gemäß der ersten Variante beispielsweise gegen eine feststehende Rückwand des Gehäuses anschlägt, ist eine sehr präzise Festlegung der absoluten Lage des Spülgutträgers relativ zum Gehäuse erreicht. Die Federeinrichtung auf der gegenüberliegenden Seite stellt dabei sicher, dass der Trägerkörper durch Federkraft immer in diese Endlage drängt.

Die zweite Variante mit an beiden Querseiten vorgesehenen Federeinrichtungen sorgt für eine Art schwimmende Lagerung des Trägerkörpers im Spülraum, wobei der Trägerkörper durch die Federkräfte immer in eine Mittellage zwischen den beiden Türen der Spülmaschine drängt. Unter der Annahme, dass die Dichtungen zwischen den Türen und dem Gehäuse auf beiden Seiten in etwa gleichmäßig verschleißen, ist überraschend einfach sichergestellt, dass der Trägerkörper ohne Fixierung am Gehäuse selbst dennoch sehr präzise immer die gleiche Lage innerhalb des Spülraums einhält.

Zur Erreichung der zuvor beschriebenen Vorteile ist vorgesehen, dass die Federeinrichtung einen sich zumindest auch in Einschubrichtung erstreckenden Federweg aufweist. Die Federeinrichtung kann eine Schraubenfeder, eine Biegefeder, eine Luftfeder, eine Elastomerfeder und/oder dergleichen aufweisen. Entscheidend ist, dass die Federeinrichtung elastisch rückstellend ausgebildet ist, das heißt, dass sie unter Belastung nachgibt und nach Entlastung in ihre ursprüngliche Gestalt zurückkehrt.

Gemäß einer Weiterbildung der Erfindung weist die Federeinrichtung eine stirnseitig an einer Querseite des Trägerkörpers angeordnete Anschlagplatte auf, welche um eine rechtwinklig zur Einschubrichtung ausgerichtete Achse drehbar gelagert ist, wobei ein Federelement zwischen der Querseite des Trägerkörpers und dem Hebel angeordnet ist. Die Anschlagplatte kann dem Grunde nach eine beliebige Erstreckung aufweisen. Sie kann sich über die gesamte Breite der Querseite des Trägerkörpers erstrecken. Zwischen der Anschlagplatte und der Querseite des Trägerkörpers ist das Federelement eingespannt. Es kann sich hierbei in vorteilhafter Weise um ein vergleichsweise kleines und beispielsweise nur punktuell wirkendes Federelement handeln, da durch die Anschlagplatte bereits ein großflächiger Kontakt mit beispielsweise einer Rückwand oder einer Tür der Spülmaschine sichergestellt ist.

Wie bereits ausgeführt, eignet sich ein zuvor beschriebener Spülgutträger insbesondere dann, wenn an einer Längsseite des Trägerkörpers ein mit einer spülmaschinenseitigen Kupplungsvorrichtung verbindbares Anschlussstück zur Verteilung von Spül-, Desinfektionsflüssigkeit und/oder Trocknungsluft angeordnet ist. Je besser eine bestimmungsgemäße Lage des Spülgutträgers bzw. Trägerkörpers mit Anschlussstück erreicht werden kann, desto sicherer und zuverlässiger kann die Verbindung zwischen der Kupplungsvorrichtung und dem Anschlussstück ausgebildet werden.

Spülmaschinenseitig ist vorgesehen, dass der zuvor beschriebene Spülgutträger in der Einschubrichtung mit der Federeinrichtung im geschlossenen Spülraum anschlägt. Dabei ist vorgesehen, dass der Spülgutträger in der Einschubrichtung beidseitig mit jeweils einer Federeinrichtung im geschlossenen Spülraum anschlägt.

Letztgenannte Ausbildung ist besonders dann von Vorteil, wenn das Gehäuse zwei jeweils mit einer Tür verschließbare Beschickungsöffnungen aufweist, welche an einander gegenüberliegenden Seiten des Gehäuses vorgesehen sind.

Die Länge des Spülgutträgers, das heißt die Länge des Trägerkörpers ergänzt durch den in Einschubrichtung stirnseitig vorstehenden Teil der Federeinrichtungen, wird erfindungsgemäß derart gewählt, dass der Spülgutträger mit der Federeinrichtung und der gegenüberliegenden Querseite beziehungsweise mit den an beiden Querseiten angeordneten Federeinrichtungen im geschlossenen Spülraum anschlägt. Dies insbesondere unter Auslenkung der Federeinrichtungen.

Vorteile und Merkmale der Erfindung ergeben sich auch anhand der nachfolgenden Figurenbeschreibung. Es zeigen:
- Fig. 1: eine Ausführungsform einer erfindungsgemäßen Spülmaschine in einer Querschnittsansicht;
- Fig. 2: ein Detail der Spülmaschine gemäß Fig. 1 in einem ersten Verschleißzustand; und
- Fig. 3: das Detail gemäß Fig. 2 in einem zweiten Verschleißzustand.

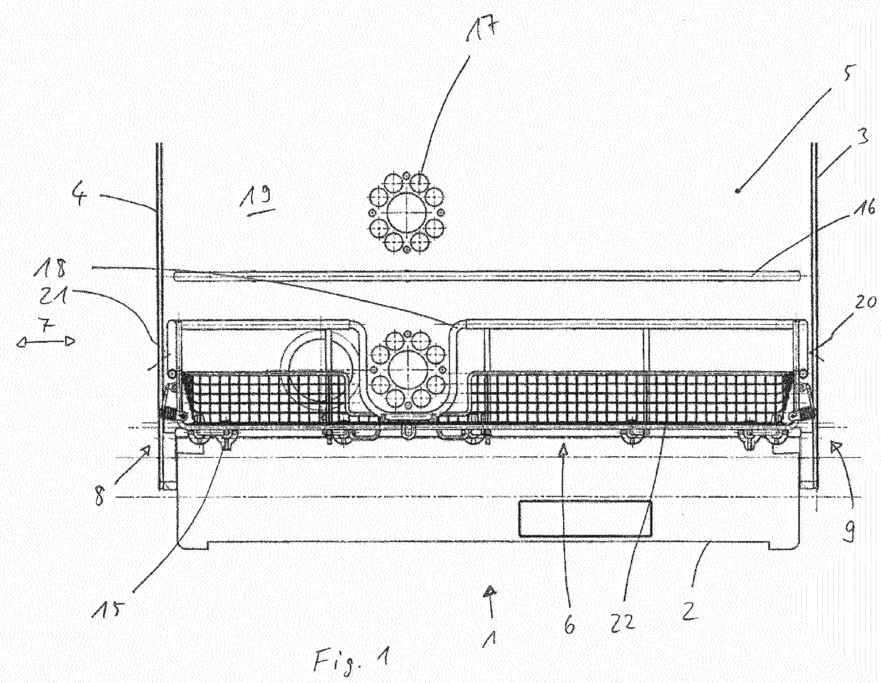

Fig. 1 zeigt eine Ausführungsform einer erfindungsgemäßen Spülmaschine 1. Dargestellt ist ein vertikaler Querschnitt in einer Richtung rechtwinklig zur einen Beschickungsöffnung der Spülmaschine 1.

Die Spülmaschine 1 verfügt über ein Gehäuse 2. Das Gehäuse 2 weist zwei einander gegenüberliegende Beschickungsöffnungen auf, welche jeweils mit einer Tür 3, 4 verschlossen sind. Bei der Spülmaschine 1 handelt es sich folglich um eine sogenannte Durchschubmaschine, bei welcher Spülgut von einer ersten Seite in die Spülmaschine eingebracht werden kann und an einer zweiten, gegenüberliegenden Seite entnommen werden kann.

Das Gehäuse 2 stellt einen Spülraum 5 bereit. Der Spülraum 5 ist in der in Fig. 1 gezeigten Konfiguration geschlossen ausgebildet, da die beiden Türen 3, 4 die Beschickungsöffnungen verschließen.

Im Spülraum 5 ist ein Spülgutträger 6 angeordnet. Der Spülgutträger 6 weist Gleitelemente 15 auf, mit welcher er an Schienen 16 innerhalb des Spülraumes 5 geführt bewegt werden kann. Auf diese Weise lässt sich der Spülgutträger 6 in Einschubrichtung 7 in den Spülraum 5 einschieben beziehungsweise herausziehen.

Im Spülraum 5 sind vorliegend drei Schienen 16 angeordnet, so dass insgesamt drei mögliche Positionen für den Spülgutträger 6 vorgesehen sind. In jeder dieser drei Positionen ist an einer Seitenwand 19 des Gehäuses 2 eine Kupplungsvorrichtung 17 vorgesehen. Diese kann mit einem nicht dargestellten Anschlussstück des Spülgutträgers 6 verbunden werden. Für das Anschlussstück ist am Spülgutträger 6 eine Anschlussstückaufnahme 18 vorgesehen.

Der Spülgutträger 6 weist einen Trägerkörper 22 auf. Dieser weist in Einschubrichtung 7 verlaufende Längsseiten und quer dazu verlaufende Querseiten 20, 21 auf. An den Querseiten 20, 21 sind stirnseitig Federeinrichtungen 8, 9 angeordnet. Mit den beiden Federeinrichtungen 8, 9 schlägt der Spülgutträger 6 an den geschlossenen Türen 3, 4 an. Auf diese Weise ist der Spülgutträger 6 im Spülraum 5 eingespannt. Eine Bewegung ist nur entgegen der Federkraft der Federeinrichtungen 8, 9 möglich, wobei der Spülgutträger 6 aufgrund der Federeinrichtungen 8, 9 immer in eine Ruhelage zurückdrängt. Diese ist vorliegende mittig zwischen den Türen 3, 4, da die beiden Federeinrichtungen 8, 9 gleich ausgebildet sind.

Auf diese Weise wird eine Art schwimmende Lagerung des Spülgutträgers 6 erreicht. Dies ist vorteilhaft, um etwaige Toleranzen, beispielsweise infolge Verschleiß, auszugleichen.

Ein Beispiel für eine solche Toleranz infolge Verschleiß stellt die Position der Türen 3, 4 relativ zum Gehäuse 2 dar. Zwischen den Türen 3, 4 und dem Gehäuse 2 ist ein nicht dargestelltes Dichtelement angeordnet. Dieses verschleißt im Laufe der Lebensdauer, wobei ein Austausch aufwendig und teuer ist. Ebenso ist eine verschleißfestere Ausgestaltung des Dichtelements aufwendig und teuer. Der Verschleiß führt dazu, dass die Türen 3, 4 sich in der geschlossenen Lage dem Gehäuse 2 immer weiter annähern. Der Abstand zwischen den Türen 3, 4 verringert sich daher im Laufe der Lebensdauer.

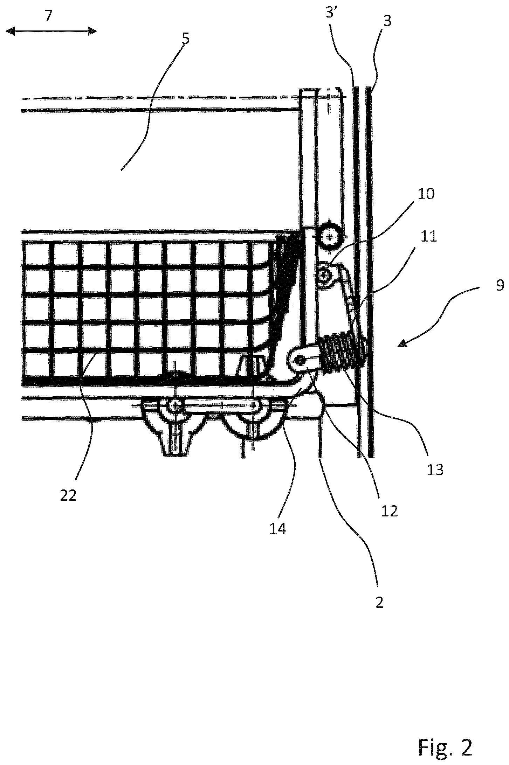

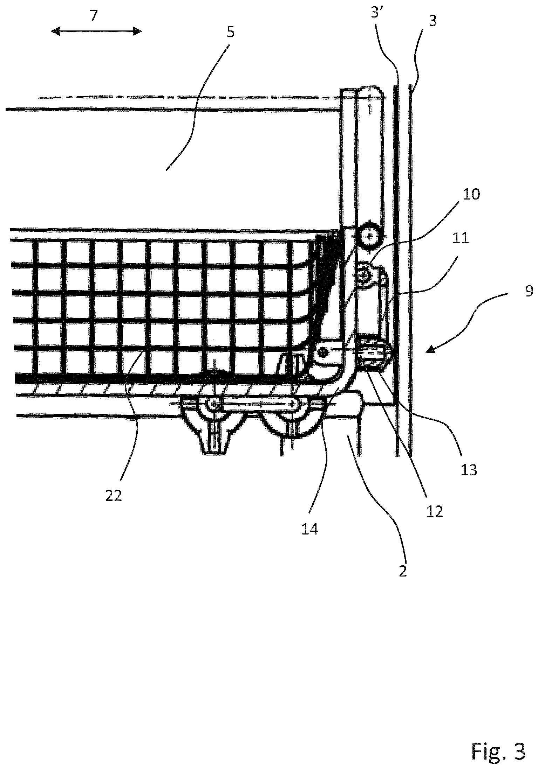

In Fig. 2 ist ein Detail der Spülmaschine 1 dargestellt, nämlich der Kontakt der in der Figur rechten Federeinrichtung 9 mit der Tür 3. Die Tür 3 weist einen vergleichsweise großen Abstand zum Gehäuse 2 auf. Dementsprechend ist die Federeinrichtung 9 vergleichsweise entspannt und weit ausgelenkt. Ein zweiter Verschleißzustand ist hingegen in Fig. 3 dargestellt. Die zwischen der Tür 3' und dem Gehäuse 2 befindliche Dichtung ist infolge Verschleiß dünner geworden. Die Tür 3' befindet sich nunmehr in der mit 3' gekennzeichneten Lage, in welcher der Abstand zwischen der Tür 3' und dem Gehäuse 2 geringer ist. Bei herkömmlichen Spülmaschinen wird die Länge des Spülgutträgers derart gewählt, dass ein Sicherheitsabstand zwischen den Querseiten des Spülgutträgers und den Türen verbleibt. Das erfindungsgemäße Prinzip zeichnet sich hingegen dadurch aus, dass die Federeinrichtungen 8, 9 stets in Kontakt mit den Türen 3, 4 bleiben. In Fig. 3 ist daher die Federeinrichtung 9 in einer zweiten Konfiguration gezeigt, in welcher die Feder komprimiert ist und dadurch den verringerten Abstand zwischen den Türen 3, 4 ausgleicht.

Der Abstand zwischen den Türen 3, 4 verringert sich folglich. Unter der Annahme, dass der Verschleißzustand der Dichtungen an den Türen 3, 4 in etwa gleich ist, führt dies jedoch nur zu einer beidseitig stärkeren Kompression der Federeinrichtungen 8, 9 des Spülgutträgers 6. Die relative Lage des Trägerkörpers 22 im Gehäuse 2 bleibt hingegen im Wesentlichen konstant. Dies bewirkt in vorteilhafter Weise, dass stets eine sichere Verbindung zwischen dem nicht dargestellten Anschlussstück des Spülgutträgers 6 und der Kupplungsvorrichtung 17 ausgebildet werden kann.

Die Federeinrichtung 8, 9 ist im vorliegenden Ausführungsbeispiel beispielhaft dadurch ausgebildet, dass eine Anschlagplatte 11 vorgesehen ist, welche um ein Drehgelenk 10 drehbar gelagert ist. Das Drehgelenk 10 erstreckt sich parallel zur Querseite 20 des Trägerkörpers 22. Die Anschlagplatte 11 kann sich über die gesamte Breite der Querseite 20 erstrecken. An einem um das Drehgelenk 10 beweglichen Ende ist die Anschlagplatte 11 mit einem Kolben 12 verbunden. Der Kolben 12 ist insbesondere in Einschubrichtung 7 beweglich gelagert, und zwar relativ zum Trägerkörper 22. Hierzu stellt der Trägerkörper 22 eine an einem Steg 14 vorgesehene Aufnahme für den Kolben 12 bereit.

Auf den Kolben 12 ist eine Schraubenfeder 13 aufgesteckt. Diese ist derart angeordnet, dass sie zwischen der Rückseite der Anschlagplatte 11 einerseits und dem Steg 14 eingespannt ist. Das Anschlagen der Anschlagplatte 11 an der Tür 3 führt folglich dazu, dass die Schraubenfeder 13 elastisch gestaucht wird.

### Bezugszeichen

- 1: Spülmaschine
- 2: Gehäuse
- 3, 3': Tür
- 4: Tür
- 5: Spülraum
- 6: Spülgutträger
- 7: Einschubrichtung
- 8: Federeinrichtung
- 9: Federeinrichtung
- 10: Drehgelenk
- 11: Anschlagplatte
- 12: Kolben
- 13: Schraubenfeder
- 14: Steg
- 15: Gleitelement
- 16: Schiene
- 17: Kupplungsvorrichtung
- 18: Anschlussstückaufnahme
- 19: Seitenwand
- 20: Querseite
- 21: Querseite
- 22: Trägerkörper

## Patentansprüche

1. Spülmaschine (1), insbesondere Reinigungs- und/oder Desinfektionsautomat für schlauchförmiges Reinigungsgut, mit einem zumindest eine Beschickungsöffnung aufweisenden Gehäuse (2) und einer die Beschickungsöffnung verschließenden Tür (3, 4), wobei das Gehäuse (2) und die geschlossene Tür (3, 4) zusammen einen geschlossenen Spülraum (5) bereitstellen, sowie mit einem Spülgutträger (6) zum Einschub in die Spülmaschine (1), welcher Spülgutträger (6) in einer Einschubrichtung (7) relativ zum Gehäuse (2) bewegbar ist und einen Trägerkörper (22) zur Aufnahme von Spülgut aufweist, welcher sich in Einschubrichtung (7) erstreckende Längsseiten und rechtwinklig zur Einschubrichtung (7) erstreckende Querseiten (20, 21) aufweist,
**dadurch gekennzeichnet,**
**dass** an beiden einander in Einschubrichtung (7) gegenüberliegenden Querseiten (20, 21) des Trägerkörpers (22) jeweils stirnseitig eine Federeinrichtung (8, 9) angeordnet ist, und dass eine Federeinrichtung (8, 9) in Einschubrichtung des Spülgutträgers (6) an einem von der Spülmaschine (1) im geschlossenen Spülraum (5) bereitgestellten Widerlager anschlägt.

2. Spülmaschine nach Anspruch 1, **dadurch gekennzeichnet, dass** der Spülgutträger (6) in der Einschubrichtung (7) beidseitig mit jeweils einer Federeinrichtung (8, 9) im geschlossenen Spülraum (5) anschlägt.

3. Spülmaschine nach einem der vorangehenden Ansprüche, **dadurch gekennzeichnet, dass** die Federeinrichtung (8, 9) einen sich zumindest auch in Einschubrichtung (7) erstreckenden Federweg aufweist.

4. Spülmaschine nach einem der vorangehenden Ansprüche, **dadurch gekennzeichnet, dass** die Federeinrichtung (8, 9) eine Schraubenfeder (13) aufweist.

5. Spülmaschine nach einem der vorangehenden Ansprüche, **dadurch gekennzeichnet, dass** die Federeinrichtung (8, 9) eine stirnseitig an einer Querseite (20, 21) des Trägerkörpers (22) angeordnete Anschlagplatte (11) aufweist, welche um eine rechtwinklig zur Einschubrichtung (7) ausgerichtete Achse drehbar gelagert ist, wobei ein Federelement zwischen der Querseite (20, 21) des Trägerkörpers (22) und der Anschlagplatte (11) angeordnet ist.

6. Spülmaschine nach einem der vorangehenden Ansprüche, **dadurch gekennzeichnet, dass** an einer Längsseite des Trägerkörpers (22) ein mit einer spülmaschinenseitigen Kupplungsvorrichtung (17) verbindbares Anschlussstück zur Verteilung von Spül-, Desinfektionsflüssigkeit und/oder Trocknungsluft angeordnet ist.

7. Spülmaschine nach einem der vorangehenden Ansprüche, **dadurch gekennzeichnet, dass** das Gehäuse (2) zwei jeweils mit einer Tür (3, 4) verschließbare Beschickungsöffnungen aufweist, welche an einander gegenüberliegenden Seiten des Gehäuses (2) vorgesehen sind.

8. Spülmaschine nach einem der vorangehenden Ansprüche, **dadurch gekennzeichnet, dass** die Länge des Spülgutträgers (6) derart gewählt ist, dass der Spülgutträger (6) mit der Federeinrichtung (8, 9) und der gegenüberliegenden Querseite (20, 21) beziehungsweise mit den an beiden Querseiten (20, 21) angeordneten Federeinrichtungen (8, 9) im geschlossenen Spülraum (5) anschlägt, insbesondere unter Auslenkung der Federeinrichtung(en) (8, 9) im geschlossenen Spülraum (5) anschlägt.

## Claims

1. Dishwasher (1), in particular a cleaning and/or disinfection machine for tubular items to be cleaned, comprising a housing (2) that comprises at least one loading opening and a door (3, 4) that closes the loading opening, the housing (2) and the closed door (3, 4) together providing a closed washing chamber (5), and comprising a carrier (6) for items to be washed that is to be inserted into the dishwasher (1), which carrier (6) for items to be washed can be moved in a insertion direction (7) relative to the housing (2) and comprises a support element (22) for holding items to washed, which support element has longitudinal sides that extend in the insertion direction (7) and transverse sides (20, 21) that extend at a right angle to the insertion direction (7),
**characterised in that**
a spring device (8, 9) is arranged on each end face on both transverse sides (20, 21) of the support element (22), which transverse sides are opposite one another in the insertion direction (7), and **in that**, in the insertion direction of the carrier (6) for items to be washed, a spring device (8, 9) strikes an abutment provided by the dishwasher (1) in the closed washing chamber (5).

2. Dishwasher according to claim 1, **characterised in that**, in the insertion direction (7), both sides of the carrier (6) for items to be washed bounce, respectively, against a spring device (8, 9) in the closed washing chamber (5).

3. Dishwasher according to any of the preceding claims, **characterised in that** the spring device (8, 9) has a spring path that also extends at least in the insertion direction (7).

4. Dishwasher according to any of the preceding claims, **characterised in that** the spring device (8, 9) comprises a helical spring (13).

5. Dishwasher according to any of the preceding claims, **characterised in that** the spring device (8, 9) comprises a stop plate (11) arranged on the end face of a transverse side (20, 21) of the support element (22), which stop plate is mounted so as to be rotatable about an axis oriented at a right angle to the insertion direction (7), a spring element being arranged between the transverse side (20, 21) of the support element (22) and the stop plate (11).

6. Dishwasher according to any of the preceding claims, **characterised in that** a connection piece, which can be connected to a coupling device (17) on the dishwasher, for distributing washing liquid, disinfecting liquid and/or drying air, is arranged on a longitudinal side of the support body (22).

7. Dishwasher according to any of the preceding claims, **characterised in that** the housing (2) comprises two loading openings, each of which can be closed by a door (3, 4), which loading openings are provided on mutually opposing sides of the housing (2).

8. Dishwasher according to any of the preceding claims, **characterised in that** the length of the carrier (6) for items to be washed is selected such that the carrier (6) for items to be washed bounces against the spring device (8, 9) and the opposing transverse side (20, 21) and/or bounces against the spring devices (8, 9) arranged on both transverse sides (20, 21) in the closed washing chamber (5), in particular by deflecting the spring device(s) (8, 9) in the closed washing chamber (5).

## Revendications

1. Lave-vaisselle (1), en particulier machine automatique de nettoyage et/ou de désinfection pour articles tubulaires à nettoyer, comprenant un carter (2) comportant au moins une ouverture de chargement et une porte (3, 4) fermant l'ouverture de chargement, le carter (2) et la porte fermée (3, 4) produisant conjointement un espace de lavage fermé (5), et un porte-vaisselle (6) destiné à être inséré dans le lave-vaisselle (1), lequel porte-vaisselle (6) est déplaçable par rapport au carter (2) dans une direction d'insertion (7) et comprend un corps de support (22) servant à recevoir des articles à laver, lequel corps de support comprend des côtés longitudinaux s'étendant dans la direction d'insertion (7) et des côtés transversaux (20, 21) s'étendant perpendiculairement à la direction d'insertion (7),
**caractérisé en ce**
**qu'**un dispositif ressort (8, 9) est disposé respectivement de manière frontale au niveau des deux côtés transversaux (20, 21), opposés l'un à l'autre dans la direction d'insertion (7), du corps de support (22), et **en ce qu'**un dispositif ressort (8, 9) vient en butée, dans la direction d'insertion du porte-vaisselle (6), contre une butée fournie par le lave-vaisselle (1) dans l'espace de lavage fermé (5).

2. Lave-vaisselle selon la revendication 1, **caractérisé en ce que** le porte-vaisselle (6) vient en butée dans l'espace de lavage fermé (5), dans la direction d'insertion (7), des deux côtés respectivement à l'aide d'un dispositif ressort (8, 9).

3. Lave-vaisselle selon l'une des revendications précédentes, **caractérisé en ce que** le dispositif ressort (8, 9) présente une course de ressort s'étendant au moins également dans la direction d'insertion (7).

4. Lave-vaisselle selon l'une des revendications précédentes, **caractérisé en ce que** le dispositif ressort (8, 9) comprend un ressort hélicoïdal (13).

5. Lave-vaisselle selon l'une des revendications précédentes, **caractérisé en ce que** le dispositif ressort (8, 9) comprend une plaque de butée (11) disposée du côté frontal sur un côté transversal (20, 21) du corps de support (22), laquelle plaque de butée est montée de manière à pouvoir tourner autour d'un axe orienté perpendiculairement à la direction d'insertion (7), un élément ressort étant disposé entre le côté transversal (20, 21) du corps de support (22) et la plaque de butée (11).

6. Lave-vaisselle selon l'une des revendications précédentes, **caractérisé en ce qu'**une pièce de raccordement pouvant être reliée à un dispositif d'accouplement (17) côté lave-vaisselle et servant à distribuer du liquide de lavage, du liquide de désinfection et/ou de l'air de séchage est disposée sur un côté longitudinal du corps de support (22).

7. Lave-vaisselle selon l'une des revendications précédentes, **caractérisé en ce que** le carter (2) comprend deux ouvertures de chargement pouvant être fermées respectivement à l'aide d'une porte (3, 4), lesquelles ouvertures de chargement sont prévues sur des côtés opposés l'un à l'autre du carter (2).

8. Lave-vaisselle selon l'une des revendications précédentes, **caractérisé en ce que** la longueur du porte-vaisselle (6) est sélectionnée de telle sorte que le porte-vaisselle (6) vient en butée dans l'espace de lavage fermé (5) à l'aide du dispositif ressort (8, 9) et du côté transversal opposé (20, 21), respectivement à l'aide des dispositifs ressorts (8, 9) disposés au niveau des deux côtés transversaux (20, 21), en particulier vient en butée dans l'espace de lavage fermé (5) en déviant le(s) dispositif(s) ressort(s) (8, 9).
